# EUROPÄISCHE PATENTSCHRIFT

(11) **EP 2 452 028 B1**
(45) Veröffentlichungstag und Bekanntmachung des Hinweises auf die Patenterteilung: **17.08.2016**
(21) Anmeldenummer: 10719338.5
(22) Anmeldetag: 10.05.2010
(51) Int. Cl.: E04F 11/16, E04F 19/06

(54) **VORRICHTUNG MIT EINER PROFILKONSTRUKTION ZUM FESTLEGEN DES RANDS EINES BODENBELAGS**
DEVICE WITH A PROFILED STRUCTURE FOR FIXING THE EDGE OF A FLOOR COVERING
DISPOSITIF AVEC UNE CONSTRUCTION PROFILÉE POUR LA FIXATION DU BORD D'UN DALLAGE

(30) Priorität: 09.07.2009 DE 102009032673; 09.07.2009 DE 202009017769 U
(43) Veröffentlichungstag der Anmeldung: 16.05.2012
(73) Patentinhaber: Küberit Profile Systems GmbH & Co. KG, 58513 Lüdenscheid (DE)
(72) Erfinder: SONDERMANN, Frank, 57489 Drolshagen (DE)
(74) Vertreter: Peter, Julian
(86) Internationale Anmeldenummer: PCT/EP2010/056383
(87) Internationale Veröffentlichungsnummer: WO 2011/003650

(56) Entgegenhaltungen:
- EP-A1- 0 773 335
- WO-A1-2007/048157
- WO-A2-2005/065381
- AT-U1- 10 366
- DE-A1- 10 246 968
- JP-A- 52 116 612
- JP-A- 2005 207 063
- JP-U- 55 121 832
- JP-U- 56 038 687
- US-A1- 2006 201 093

## Beschreibung

Die Erfindung betrifft eine Vorrichtung gemäß dem Oberbegriff des Anspruchs 1.

Im Stand der Technik sind Profilkonstruktionen bekannt, die an den Rändern eines Bodenbelags vorgesehen sind, um den Bodenbelag dagegen zu schützen, dass er sich am Rand vom Boden lösen kann. Der Rand des Bodenbelags ist einer besonderen Belastung ausgesetzt, da er ungeschützt leicht Angriffsfläche zum Abstoßen bieten kann. Es werden daher an kritischen Stellen Profilkonstruktionen verwendet, die beispielsweise an Treppenkanten oder an Übergängen zu anderen Bodenbelägen eingesetzt werden.

Aus der EP 773 335 ist ein Treppenkantenprofil bekannt, das aus zwei Elementen besteht, nämlich einem Trittwinkelprofil und einem Basisprofil. An dem Trittwinkelprofil ist ein Trittschenkel ausgebildet, der an seinem freien Ende einen Abdeckflügel für einen Treppenbelag aufweist. Das Trittwinkelprofil wird auf das auf der Treppe im Kantenbereich festlegbare Basisprofil aufgesetzt und besitzt einen im rechten Winkel an den Trittschenkel nach unten ragenden Anschlagschenkel sowie im Abstand dazu einen zusätzlichen Steg. Der Treppenbelag wird nach dem Aufsetzen des Trittwinkelprofils von dem Abdeckflügel um ein vorbestimmtes Maß überlappt, so dass ein Abheben oder Abstoßen des Rands des Treppenbelags sicher verhindert wird.

Diese Vorrichtung arbeitet zufriedenstellend, insbesondere dann, wenn der Treppenbelag um ein bestimmtes Maß zusammendrückbar ist, so dass der Außenrand des Abdeckflügels etwas in den Belag eindringen kann und es so vermieden wird, dass zwischen der Belagoberfläche und dem Trittwinkelprofil ein merkbarer Stoß entsteht. Im Falle eines Parkettfußbodens oder eines Laminatbodenbelags ist ein Eindringen in den Bodenbelag nicht möglich, es sei denn, der Randbereich wäre mit einer entsprechenden Ausfräsung für den Abdeckflügel versehen. Dies ist jedoch auf der Baustelle nur unter erschwerten Bedingungen bzw gar nicht möglich. Weitere Vorrichtungen mit einer entsprechenden Profilkonstruktion sind aus den Dokumenten JP 56 038687 U. AT 10 366 U1, WO 2005/065381 A2 und US 2006/201093 A1 bekannt.

JP 55 121832 U offenbart eine Vorrichtung gemäß dem Oberbegriff des Anspruchs 1.

Der Erfindung liegt die Aufgabe zugrunde, eine Vorrichtung zum Festlegen eines Bodenbelags an einer Profilkonstruktion zu schaffen, mittels welcher ein mit einer Profilierung versehener Rand des Bodenbelags unter Vermeidung eines Anschlussstoßes sicher und dauerhaft gehalten werden kann.

Die Aufgabe wird erfindungsgemäß gelöst durch die Merkmalkombination gemäß Patentanspruch 1 und dabei unter anderem durch einen lösbar und in seiner Lage anpassbar an der Profilkonstruktion angeordneten, in Form einer Profilschiene ausgebildeten Wechseleinsatz, an dem zumindest an einem seiner beiden einander gegenüberliegenden Längsränder eine mit der Profilierung des Randes des Bodenbelages zusammenwirkende Halterung ausgebildet ist, die zumindest teilweise komplementär zur Profilierung ausgebildet ist. Dadurch, dass der Rand des Bodenbelags, der mit einer Profilierung versehen ist, die mit der komplementären Halterung zusammenwirken kann, wird eine sichere Halterung des freien Randes des Bodenbelags bereitgestellt und verhindert, dass dieser sich vom Boden abheben kann.

Bei einer vorteilhaften Ausbildung wird der Wechseleinsatz ganz oder zum Teil zwischen dem Trittschenkel und dem Basisprofil angeordnet, wobei der Wechseleinsatz bevorzugt an seinen beiden einander gegenüberliegenden Längsrändern voneinander unterschiedliche Halterungen zum Einsatz an unterschiedlichen Profilierungen von Nut - Feder - Verbindungen aufweist. Durch diese besondere Anordnung wird eine sichere und unverrückbare Positionierung des Wechseleinsatzes an dem Treppenkantenprofil bereitgestellt.

Bei einer besonders vorteilhaften Ausgestaltung ist der Wechseleinsatz an der Profilkonstruktion mittels Verrastung gehalten. Hierdurch ist es wahlfrei möglich, den Wechseleinsatz zunächst in die Profilkonstruktion einzusetzen und anschließend den Rand des Bodenbelags anzubringen oder aber zuerst eine Verbindung mit dem Randprofil einzugehen und anschließend daran den Anschluss mit der Profilkonstruktion herzustellen.

Dabei ist es vorteilhaft, wenn der Wechseleinsatz als Teil der Verrastung im Bereich der komplementären Halterung eine Verankerung mit dem Basisprofil und/oder dem Trittschenkel aufweist. Hierdurch ist es gewährleistet, dass der Bodenbelag sich gegenüber der Vorrichtung nicht verändern kann.

Erfindungsgemäß ist der Wechseleinsatz mit mindestens einer Abstützrippe versehen, die an ihrem freien Stützrand eine Rasterung aufweist, die mit einer entsprechenden Rasterung auf dem Basisprofil zusammenwirkt. Diese Abstützrippe kann das Trittwinkelprofil im Bereich des Trittschenkels oder des Abdeckflügels abstützen, so dass der Trittschenkel nicht niedergetreten werden kann. Diese Gefahr wäre gegeben, da er bei der Erfindung nicht mehr den Bodenbelag überlappt, sondern lediglich an dessen Rand anstößt und nicht mehr aufliegt. Die Verrastung bewirkt eine dauerhafte Lage - Festlegung, so dass der Wechseleinsatz nicht verrutschen kann.

Vorteilhafterweise ist an der Abstützrippe ein zur Aufnahme waagerechter Kräfte dienender Steg ausgebildet, an dem die komplementäre Halterung anschließt. Hierdurch können die auf den Bodenbelag einwirkenden Kräfte über die komplementäre Halterung und den Steg sowie der Abstützrippe in die Vorrichtung eingeleitet werden.

Als eine günstige Ausbildung kann vorgesehen sein, den Wechseleinsatz als Stranggussprofil aus Aluminium oder Kunststoff zu fertigen. Hierdurch ist eine kostengünstige Fertigung gegeben.

Die Profilierung des Rands des Bodenbelags und somit die entsprechende teilweise komplementäre Halterung kann ganz oder teilweise in einer Art Nut-Feder-Verbindung ausgestaltet sein, die mindestens einen zur Nut-Feder-Ebene vortretenden Vorsprung bzw. eine Vertiefung aufweist.

Dabei ist es günstig, wenn die komplementäre Halterung mindestens eine Halterippe oder einen Haltevorsprung aufweist, der bzw. die die Nut-Feder-Verbindung nach oben sichert und/oder den Vorsprung mindestens zum Teil hintergreift bzw. in die Vertiefung eingreift. Eine derartige Ausbildung kann mit Vorteil so geschaffen sein, dass an der mindestens einen Halterippe oder dem Haltevorsprung Sollbruchstellen zur Höhenreduzierung ausgebildet sind. Die Halterippe kann dabei im Lieferzustand eine Höhe aufweisen, die im Wesentlichen der Dicke oder einer annähernden Dicke eines üblichen Profils entspricht. Je nach besonderer Ausführung der Nut-Feder-Gestaltung kann dann diese Halterippe in ihrer Höhe so weit reduziert werden, dass sie passend in die Vertiefung bzw. hinter den Vorsprung greifen kann und somit eine sichere Verankerung darstellt. Selbst für den Fall, dass die Nut-Feder-Verbindung eine Feder in Form einer normalen vorstehenden Rippe aufweist, ist die an dem Wechseleinsatz ausgebildete Halterippe in der Lage, den Randbereich des Bodenbelags insoweit zu fixieren, als sie auf der Feder aufliegen und dabei einen nach unten gerichteten Druck ausüben kann.

Eine besonders einfache Gestaltung kann darin bestehen, dass der Wechseleinsatz im Wesentlichen U-förmig ausgebildet und ein U-Schenkel als Halterippe und der andere U-Schenkel als Abstützrippe ausgebildet ist. Beide Schenkel werden in dieser Ausführungsform durch den Steg miteinander verbunden. Günstig kann dabei sein, wenn beide Schenkel Sollbruchstellen aufweisen, so dass sie an die Höhe des Bodenbelags anpassbar sind und dadurch bei unterschiedlichen Profilkonstruktionen eingesetzt werden kann.

Schließlich kann bei einer weiteren alternativen Ausführungsform der Wechseleinsatz mit einer Bohrung versehen sein, um sie mittels eines Befestigungsmittels an einen geraden Rand eines Bodenbelags, d.h. wenn der Bodenbelag am Rand keine vorstehende Profilierung, sondern eine glatte Stirnfläche besitzt, festzulegen.

Bei einer Ausführungsform, die nicht Teil der Erfindung ist, kann der Wechseleinsatz mittels eines Schienenführungssystems an dem Basisprofil gehalten sein. Somit kann der Wechseleinsatz mit dem Basisprofil eine Vorabmontage erfahren, wobei die Handhabung erleichtert wird, da lediglich mit einem Bauteil bestehend aus Wechseleinsatz und Basisprofil umgegangen werden muss.

Hierbei ist es günstig, wenn das Schienenführungssystem eine in Querschnitt kugelförmige Schienenanordnung aufweist. Jedoch kann es auch vorteilhaft sein, eine Schwalbenschwanzverbindung für das Schienenführungssystem vorzusehen. Mittels beider Verbindungsarten kann eine einfache, leichte, schnelle sowie stabile Verbindung zwischen dem Wechseleinsatz und dem Basisprofil geschaffen werden. Der Vorteil der Kugelform gegenüber dem Schwalbenschwanz ist in der gegen Stöße robusten Form zu sehen, da keine Kante oder Ecke während der Installation beschädigt werden kann. Hingegen ist der Schwalbenschwanz einfach zu fertigen, womit eine dementsprechende Kostenreduzierung einhergeht.

Zudem kann bei der Kugelform der Wechseleinsatz unter Druck in eine komplementär ausgebildete Nut in dem Basisprofil hineingedrückt werden, sodass ein genaues Einrichten nicht erforderlich ist, da die einander zugeordneten Einzelelemente ihren passenden Sitz zwangsweise finden.

Die Erfindung bezieht sich auf die Ausführungsformen der Figuren 1 bis 9. Die Figur 10 zeigt Ausführungsformen, die nicht Teil der Erfindung sind.

Es zeigen
- Fig. 1: eine als Treppenkantenprofil ausgebildete erfindungsgemäße Vorrichtung mit eingeklicktem Bodenbelag,
- Fig. 2a - Fig. 2c: Details 1 - 3 aus Fig. 1,
- Fig. 3: ein nächstes Ausführungsbeispiel der bei einem Treppenkantenprofil angewandten Erfindung mit anderer Profilierung des Bodenbelags,
- Fig. 4: ein nächstes Ausführungsbeispiel mit einer nächsten Profilierung des Rands des Bodenbelags,
- Fig. 5: eine perspektivische Darstellung einem im Wesentlichen U-förmigen Wechseleinsatz mit Bohrung,
- Fig. 6: der Wechseleinsatz aus Fig. 5 in eingebautem Zustand bei Verwendung mit einem Bodenbelag, dessen Randkante eine gerade Stirnfläche aufweist,
- Fig. 7: eine nächste Ausführungsform mit einem liegenden, im Wesentlichen U - förmigen Wechseleinsatz,
- Fig. 8: eine Ausführungsform eines Wechseleinsatzes mit beidseitiger Profilierung für die Nut - Seite wie auch für die Feder- Seite,
- Fig. 9: eine Ausführungsform eines Wechseleinsatzes mit beidseitiger Profilierung für den Einsatz bei unterschiedlichen Profilierungstypen und
- Fig. 10: verschiedene Aufnahmen für eine Befestigung eines Wechseleinsatzes an einem Basisprofil.

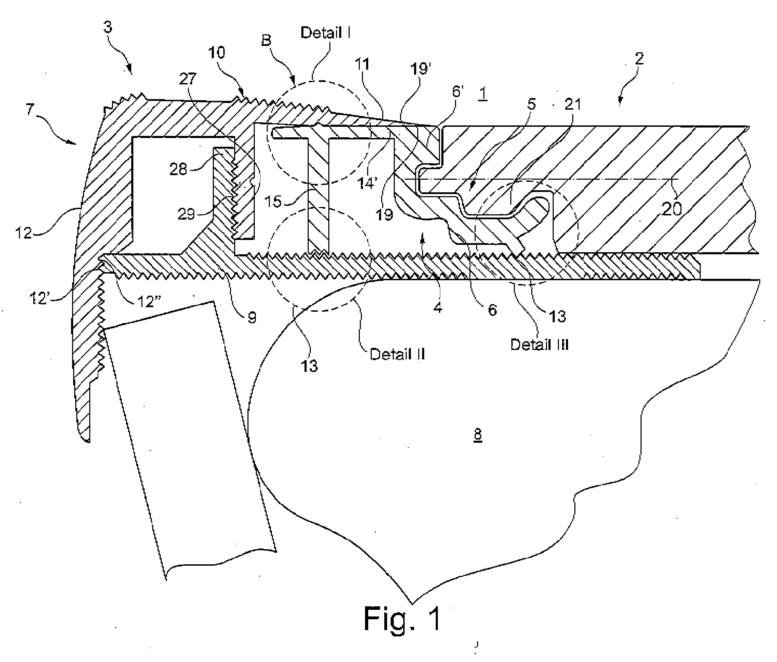

In Fig. 1 ist eine Vorrichtung zum lösbaren Festlegen des Rands 1 eines Bodenbelags 2 an einer Profilkonstruktion 3 dargestellt. Der Bodenbelag besteht aus zusammengefügten Elementen, die an ihrem Rand mit einer Profilierung im Sinne eines Nut-Feder-Systems versehen sind, wodurch eine feste Verbindung der einzelnen Elemente sichergestellt ist. Die Profilkonstruktion 3 ist bei dem dargestellten Ausführungsbeispiel ein Treppenkantenprofil, das ein Trittwinkelprofil 7 und ein auf der Trittfläche einer Treppe 8 festlegbares Basisprofil 9 aufweist. Das Trittwinkelprofil 7 besitzt einen im Wesentlichen waagerechten Trittschenkel 10 mit einem Abdeckflügel 11 und einen sich senkrecht erstreckenden Anschlagschenkel 12.

Der Anschlagschenkel 12 ist mit einer Profilierung 12' versehen, die mit einer entsprechenden Profilierung 12" an der Stirnkante des Basisprofils 9 zusammenwirkt. Das Trittwinkelprofil 7 ist an seiner Unterseite mit einer Anschlagrippe 27 ausgestattet, die mit einer Gegenrippe 28, die an den Basisprofil 9 nach oben ragt, zur Anlage kommt. Zwischen beiden Rippen ist eine Verrastung 29 ausgebildet. Das Trittwinkelprofil 7 kann somit in beliebiger Höhe an dem Basisprofil 9 eingerastet werden, so dass hierdurch eine Anpassung an die Dicke des Bodenbelags 2 vorgenommen werden kann.

Die als Treppenkantenprofil ausgebildete Profilkonstruktion 3 weist erfindungsgemäß den in Form einer Profilschiene ausgebildeten Wechseleinsatz 4 auf, der lösbar und in seiner Lage anpassbar an der Profilkonstruktion 3 angeordnet ist. An dem Wechseleinsatz 4 ist zudem eine der Profilierung 5 des Rands 1 des Bodenbelags 2 zumindest teilweise komplementäre Halterung 6 ausgebildet, die an einem ihrer beiden einander gegenüberliegenden Längsränder mit der Profilierung 5 des Rands 1 des Bodenbelags 2 zusammenwirkt.

Wie die unten näher erläuterten weiteren Ausführungsformen zeigen, kann die Halterung 6 auch derart ausgebildet sein, dass sie nur teilweise komplementär zur Profilierung 5 bzw. zur Kontur der Nut - Feder - Verbindung angepasst ist.

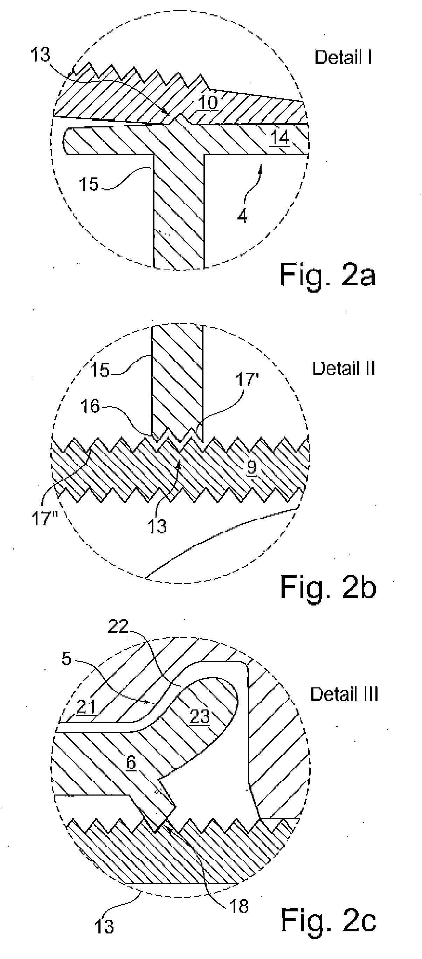

Im dargestellten Ausführungsbeispiel ist die Profilierung Bestandteil einer besonderen Nut-Feder-Verbindung 19, deren Feder 19' an dem Rand des Bodenbelags 2 nach vorne tritt. Die Profilierung 5 weist darüber hinaus einen Rücksprung auf, wodurch am Rand des Bodenbelags eine Schulter entsteht. Auf dieser Schulter ist ein Vorsprung 21 ausgebildet, der über die Nut-Feder-Ebene 20 vorsteht. Hinter dem Vorsprung 21 ist eine Vertiefung 22 ausgebildet. Die komplementäre Halterung 6 des Wechseleinsatzes 4 ist in dem in Fig. 1 dargestellten Ausführungsbeispiel so gestaltet, dass ihre Kontur dem Gegenstück der Nut-Feder-Verbindung 19 entspricht. Die Halterung 6 weist daher, wie im Detail III in Fig. 2c zu erkennen, an ihrem dem Bodenbelag zugewandten freien Ende einen Haltevorsprung 23 auf, der den Vorsprung 21 umfasst und in die Vertiefung 22 der Nut-Feder-Verbindung 19 eingreift.

Im Einzelnen besteht der Wechseleinsatz 4 aus einem Profil, das als Schiene oder in Einzelstücken Verwendung finden kann. Die an dem Profil ausgebildete Halterung setzt sich, wie insbesondere aus Fig. 1 zu erkennen, an ihren dem Treppenkantenprofil zugewandten Seiten nach oben fort und umgreift dabei die Feder 19'. Im Anschluss daran erstreckt sich im Wesentlichen in waagerechter Richtung ein Steg 14, an dem im Abstand zur Halterung 6 eine im Wesentlichen im rechten Winkel auf dem Steg 14 stehende Abstützrippe 15 ausgebildet ist.

Die Abstützrippe 15, die an ihrem freien Stützrand 16 eine Rasterung 17' aufweist, wirkt mit einer entsprechenden Rasterung 17" auf dem Basisprofil 9 zusammen.

Das Profil des Wechseleinsatzes 4 ist im dargestellten Ausführungsbeispiel gemäß Fig. 1 zum Teil zwischen dem Trittschenkel 10 und dem Basisprofil 9 angeordnet und ragt an einem der beiden einander gegenüberliegenden Längsrändern mit seinem als komplementäre Halterung 6 ausgebildeten Bereich in den Bodenbelag 2 hinein. Somit wird der bezüglich der vorstehenden Feder 19' in den Bodenbelag 2 zurückversetzte Vorsprung 21 durch Eingriff in die Vertiefung 22 hintergriffen.

Der Wechseleinsatz 4 ist mit der Profilkonstruktion 3 lösbar verbunden und daran mittels Verrastung 13 gehalten.Die Verrastung 13 greift in dem dargestellten Ausführungsbeispiel an drei Stellen an der Profilkonstruktion an. Die Stellen sind in Fig. 1 mit Detail I, Detail II und Detail III gekennzeichnet und in den entsprechenden Fig. 2a - 2c dargestellt.

In Fig. 2a ist das Detail I vergrößert wiedergegeben. Der Steg 14 des Wechseleinsatzes 4 kommt bei dieser Ausführungsform unter dem Trittschenkel 10 und dem Abdeckflügel 11 im Wesentlichen flächig zur Anlage. Im Bereich des Trittschenkels 10 weist der Steg 14 eine Verrastung 13 auf, die aus einer Rastrippe und einer entsprechend ausgebildeten Rastnut besteht. Im dargestellten Ausführungsbeispiel ist die Rastrippe auf dem Steg 14 ausgebildet und die Rastnut dementsprechend an der Unterseite des Trittschenkels. Bevorzugt befindet sich diese Verrastung im Wesentlichen über der Abstützrippe 15, die senkrecht von dem Steg 14 nach unten ragt.

Fig. 2b stellt das Detail II dar, in dem der untere Endabschnitt der Abstützrippe 15 mit der dortigen Verrastung 13 wiedergeben ist. Ein Stützrand 16 der Abstützrippe 15 ist mit einer Rasterung 17' versehen, die mit einer entsprechenden Rasterung 17" auf der Oberfläche des Basisprofils 9 zusammenwirkt. Die Abmessungen hinsichtlich der Lage sind so gewählt, dass die Verrastung 13 auf dem Steg 14 dann greift, wenn auch die Verrastung 17', 17" in Eingriff steht.

In Fig. 2c ist mit dem Detail III die dritte Verrastung 13 dargestellt. Hierbei weist der Wechseleinsatz 4 als Teil der Verrastung im Bereich der komplementären Halterung 6 eine Verankerung 18 auf, bei der Rastnuten auf dem Basisprofil einen Eingriff für einen Verankerungsvorsprung bereitstellen. Zusätzlich oder stattdessen kann der Wechseleinsatz 4 eine Verankerung 18 mit dem Trittschenkel 10 aufweisen (nicht dargestellt).

Durch die Dreipunktverankerung wird der Wechseleinsatz 4 unabhängig von seinem Eingriff mit dem Profil des Bodenbelags selbständig und sicher in Lage gehalten, wenn das Trittwinkelprofil auf dem Basisprofil eingerastet wird. Man kann dabei eine Raststellung wählen, bei der das Profil des Wechseleinsatzes 4 unter Druck zwischen dem Trittschenkel 10 und dem Basisprofil 9 eingespannt ist. Durch die Verrastung des Wechseleinsatzes 4 kann der Steg 14 waagerechte Kräfte aufnehmen, die von dem Bodenbelag über die komplementäre Halterung 6 und die Abstützrippe 15 in die Treppenkantenprofilanordnung 3 eingeleitet werden.

Bei besonderen Ausführungen kann die Rippe der Verankerung 18 eine größere Länge als dargestellt aufweisen. Die Abstützrippe 15 kann dann ebenso wie die der Verankerung 18 dienende, nach unten abstehende Rippe mit Sollbruchstellen versehen sein, wodurch eine Höhenreduzierung bzw. eine Anpassung an die Dicke des Bodenbelags ermöglicht ist.

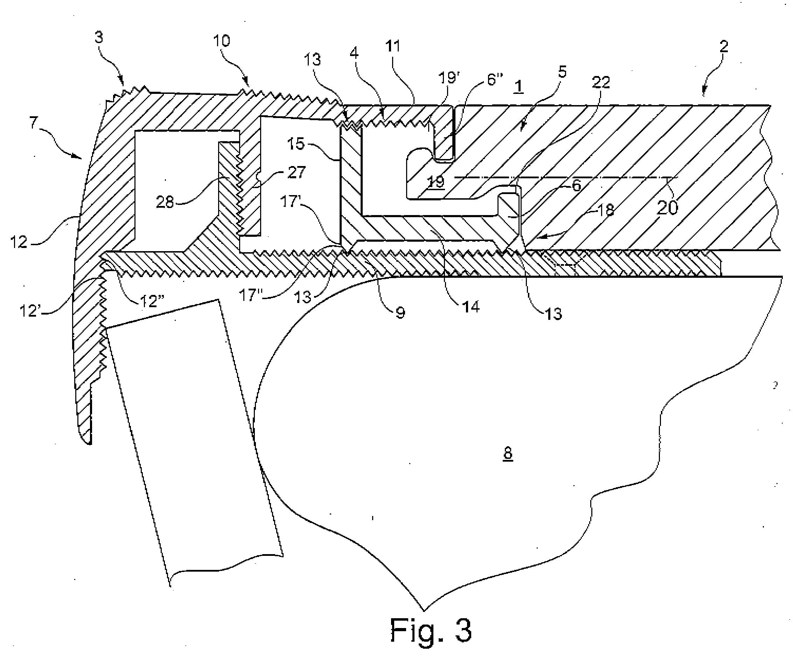

Fig. 3 zeigt ein nächstes Ausführungsbeispiel der Erfindung. Bei diesem Ausführungsbeispiel sind die Elemente, welche der gleichen Funktion dienen wie bei der Vorrichtung aus Fig.1, mit denselben Bezugszeichen versehen.

Bei dieser Ausführungsform ist der Wechseleinsatz 4 durch ein im Wesentlichen L-förmiges Profil gebildet, wobei der Steg 14 nicht unter dem Trittschenkel zur Anlage kommt, sondern im Wesentlichen unmittelbar über dem Basisprofil 9 angeordnet ist. Die Abstützrippe 15 ist wieder mit einer Verrastung 13 bzw. mit einer Rasterung 17' und 17" sowohl unter dem Trittschenkel 10 als auch auf dem Basisprofil 9 abgestützt. Der Steg 14 erstreckt sich in Richtung auf die Nut - Feder - Verbindung 19 des Rands des Bodenbelags und weist an seinem freien Ende einen im Wesentlichen senkrecht nach oben stehenden Steg als komplementäre Halterung 6 auf. Dieser Steg hintergreift eine in der Nutfederverbindung 19 vorgesehene Vertiefung 22. Unter diesem Steg der Halterung 6 ist die Verankerung 18 der Verrastung 13 ausgebildet, so dass auch bei dieser Ausführungsform eine Dreipunkthalterung gewährleistet ist. Zur Abstützung gegen den Trittschenkel 10 kann eine zusätzliche Halterippe 6" in Funktion einer Stegverlängerung auf die Feder der Nut - Feder - Verbindung 19 gelegt werden.

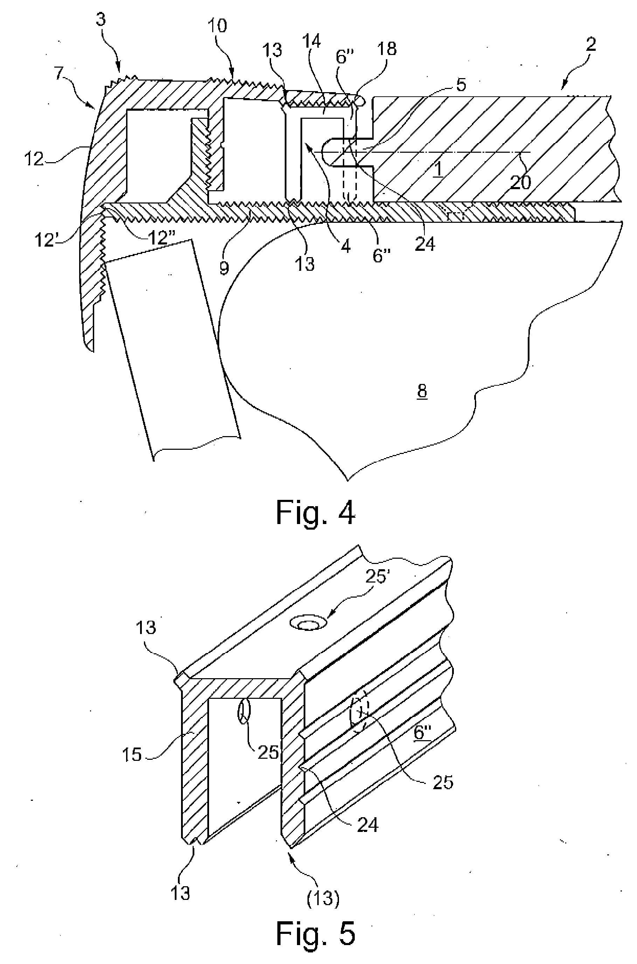

In Fig. 4 ist eine nächste Ausführungsform eines lösbaren Wechseleinsatzes 4 dargestellt. Merkmale mit denselben Bezugsziffern entsprechen hinsichtlich der Funktion den gleichwirkenden Merkmalen der Fig. 1.

Diese Ausführungsvariante macht ebenfalls Gebrauch von einem im Wesentlichen L-förmigen Profil des Wechseleinsatzes 4, wobei jedoch der den Steg 14 bildende Basisschenkel des L-Profils unterhalb des Trittschenkels 10 bzw. Abdeckflügels 11 zu liegen kommt und mittels der Verrastung bzw. Verankerung 13 an dem Trittschenkel 10 gehalten wird. Die nach unten ragende Abstützrippe 15 stützt sich mit der unteren Verrastung 13 auf dem Basisprofil 9 ab. Die komplementäre Halterung 6 ist im vorliegenden Fall als ein nach unten ragende Halte-Rippe 6" ausgebildet, der durch Abbrechen der in Fig. 4 gestrichelt dargestellten Überlänge an einer Sollbruchstelle 24 auf das geeignete Maß reduziert ist. Der Steg kommt dabei an der Profilierung 5 der Nut-Feder-Anordnung zur Anlage und verhindert ein Abheben des Belags.

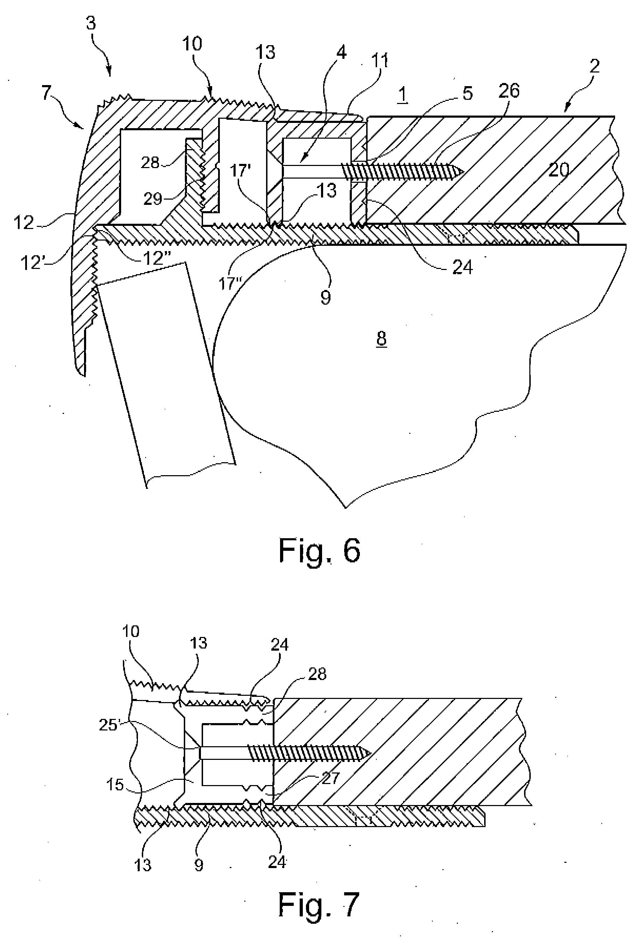

Die Fig. 5 und 6 zeigen ein weiteres Ausführungsbeispiel der Erfindung. Merkmale mit denselben Bezugsziffern entsprechen denjenigen in Fig.1.

Das in dem Ausführungsbeispiel gemäß Fig. 5 dargestellte Profil des Wechseleinsatzes 4 ist bei der hier beschriebenen Form im Wesentlichen U-förmig bzw. als U-Profil ausgebildet, wobei ein U-Schenkel als Halte-Rippe und der andere U-Schenkel als Abstützrippe 15 ausgebildet ist. Die nach unten ragende HalteRippe 6" der Halterung 6 weist in diesem Fall die gleiche Länge auf wie die Abstützrippe 15. Die Verankerung des Wechseleinsatzes am Treppenkantenprofil erfolgt ebenfalls wieder über die Verrastung 13, die an den Kanten des U-Profils ausgebildet sind, die jeweils mit entsprechenden Rastrippen unter dem Trittschenkel 10 bzw. demAbdeckflügel 11 und auf dem Basisprofil 9 zusammenwirken. Zusätzlich ist jedoch bei diesem Profil eine beide Schenkel des U-Profils durchdringende Bohrung 25 ausgebildet, die der Aufnahme einer Schraube dient, mittels welcher der Wechseleinsatz an der Stirnfläche des geraden Rands des Bodenbelags festlegbar ist.

Insbesondere findet diese Ausführungsform, wie in Fig. 6 dargestellt, Anwendung bei solchen Rändern 1, deren Seitenfläche nicht mit einer Profilierung versehen ist. Ein solcher Wechseleinsatz 4 ist auch leicht an unterschiedliche Höhen des Bodenbelags 2 anpassbar, da seine nach unten ragenden USchenkel, die über den Steg 14 miteinander verbunden sind, nach Bedarf an den Sollbruchstellen 24 auf die passende Höhe gebracht werden können.

Somit ist diese Ausführungsform der Halterung sowohl geeignet, einen Bodenbelag mit glatter Randfläche zu halten, ohne dass der Abdeckflügel 11 eine Stoßkante bildend auf dem Bodenbelag aufgelegt werden müsste, als auch, gemäß Fig. 4, geeignet zur Verwendung bei profilierten Nut - Feder - Verbindungsrändern, wobei dann der dem Bodenbelag zugewandte Halterippe 6" an den Sollbruchstellen 24 auf das geeignete Maß reduziert wird, um die Auflage bzw. Anlage einer entsprechenden Feder auf der reduzierten Halterippe 6" zu ermöglichen.

Es versteht sich, dass eine solche Verschraubung auch bei einer Verbindung gemäß Fig. 4 zum Einsatz kommen kann, wobei gegebenenfalls die vorstehende Feder im Bereich der Schraube entfernt werden kann.

In Fig. 5 ist eine weitere Bohrung 25' dargestellt, die zum Einsatz kommen kann, wenn das U- Profil nicht stehend, sondern liegend eingesetzt wird, vergl. Fig. 7. Die Verrastung 13 ist so ausgebildet, dass sie auch in liegender Stellung in entsprechende Rastnuten unter dem Trittschenkel 10 und auf dem Basisprofil 9 eingreifen kann. Bei dieser Ausführungsform können die waagerechten Schenkel des U - Profils die Nut - Feder - Ausbildung übergreifen und mit beiden oder nur mit einem Schenkel am Rand des Bodenbelags anschlagen.

Die in Fig. 7 dargestellte Verbindung macht während des Einsatzes Gebrauch der in Fig.5 dargestellten Ausführungsform mit der Bohrung 25', die an der Basis des U-förmigen Wechseleinsatzes im Wesentlichen in der Symmetrieebene vorgesehen ist. Die Schenkel 27 und 28 umgreifen eine Feder der Nut - Feder - Verbindung und kommen an der eigentlichen Seitenfläche des Rands des Bodenbelags zur Anlage, wobei auch die Breite der Feder durch Verkürzen der Schenkel berücksichtigt werden kann.

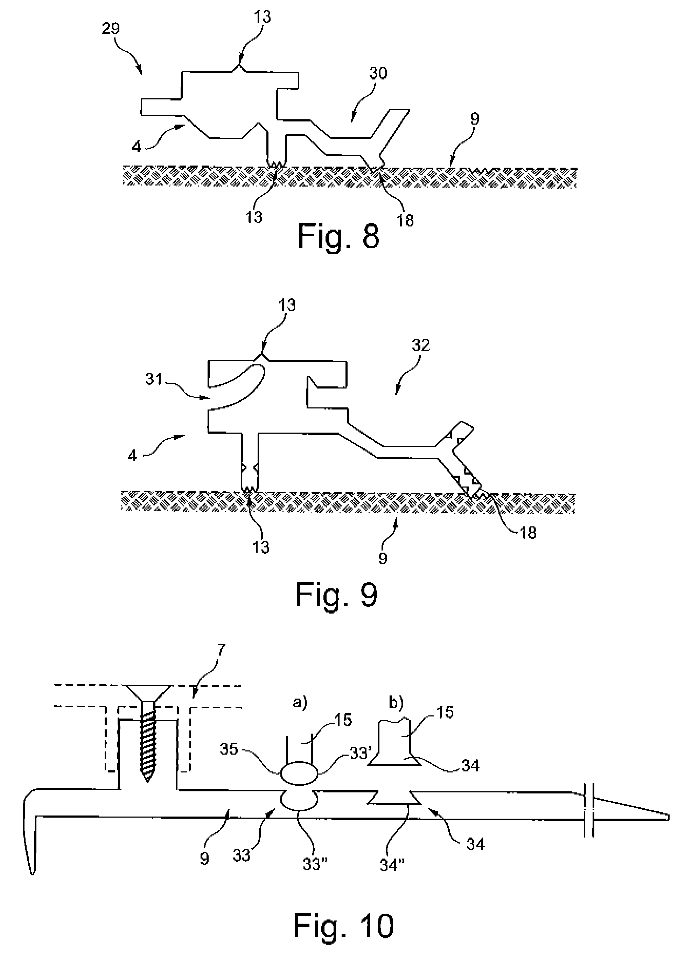

In Fig. 8 ist eine weitere Ausführungsform eines Wechseleinsatzes 4 dargestellt. Bei dieser Ausführungsform eines Wechseleinsatzes ist eine Profilierung für die Nut - Seite wie auch für die Feder - Seite jeweils an einer Längsseite eines den Wechseleinsatz bildenden Profils ausgeformt. Somit kann der Wechseleinsatz unabhängig von dem Charakter der Profilierung eingesetzt werden, ohne dass der Handwerker darauf achten müsste, ob die Nut - Seite oder die Feder - Seite an dem Treppenkantenprofil anliegt.

Fig. 9 zeigt eine Ausführungsform eines Wechseleinsatzes 4 mit beidseitiger Profilierung für den Einsatz bei unterschiedlichen Profilierungstypen 31 und 32, die gegebenenfalls bei unterschiedlichen Arten von Bodenbelägen vorhanden sind.

Schließlich sind in Fig. 10 beispielhaft verschiedene Schienenführungssysteme 33, 34 , die nicht Teil der Erfindung sind, für eine Befestigung eines Wechseleinsatzes 4 an einem Basisprofil dargestellt. Ein solches Schienenführungssystem tritt an die Stelle einer Verrastung oder Verankerung.

Die Aufnahme gemäß Beispiel a) weist an der Abstützrippe 15 eine im Wesentlichen teilzylindrischen Rastvorsprung 33' auf, der unter Druck in die komplementär ausgebildete Nut 33" in dem Basisprofil 9 hineingedrückt wird. Bei entsprechender enger Ausbildung der Einschnürung 35 ist auch die Möglichkeit gegeben, den Wechseleinsatz von der Seite in das Basisprofil einzuschieben.

Die Ausführungsform gemäß Beispiel b) sieht eine Schwalbenschwanz - Verbindung 34 vor, bei der in dem Basisprofil 9 eine Schwalbenschwanz - Führung 34" und an der Abstützrippe 15 ein Schwalbenschwanz 34' ausgebildet sind. In beiden Ausführungsformen erfolgt eine sichere und stets lagerichtige Anordnung des Wechseleinsatzes.

Es versteht sich, dass die Ausführungsbeispiele hinsichtlich ihrer in den Figuren dargestellten Dimensionen nicht zwingend den tatsächlichen Abmaßen entsprechen müssen. So ist z. B. bei den Ausführungsbeispielen der Figuren 8 und 9 dafür Sorge zu tragen, das die Maße des Wechseleinsatzes hinreichend den Platz unter dem Trittschenkel berücksichtigen.

Eine Ausführungsform, wobei die in den Figuren 1 bis 4 dargestellte Profilkonstruktion nicht auf eine Rastverbindung beschränkt ist, sondern auch solche Konstruktionen umfasst, bei von einer Schraubverbindung zwischen dem Trittwinkel 7 und dem Basisprofil 9 verwendet wird, wie in Fig.10 dargestellt, ist nicht Teil der Erfindung.

### Bezugszeichenliste

1. Rand
2. Bodenbelag
3. Profilkonstruktion
4. Wechseleinsatz
5. Profilierung
6. komplementäre Halterung
7. Trittwinkelprofil
8. Treppe
9. Basisprofil
10. Trittschenkel
11.Abdeckflügel
12.Anschlagschenkel
13.Verrastung
14. Steg
15.Abstützrippe
16. Stützrand
17.Rasterung (17', 17")
18.Verankerung
19. Nut - Feder - Verbindung
19' Feder
20. Nut - Feder - Ebene
21.Vorsprung
22.Vertiefung
23. Halterippe
24.Sollbruchstellen
25. Bohrung
26. Befestigungsmittel
27.Anschlagrippe
28.Gegenrippe
29.Verrastung

## Patentansprüche

1. Vorrichtung umfassend
- eine Profilkonstruktion ausgebildet als ein Treppenkantenprofil (3) mit einem Trittwinkelprofil (7) und einem auf einer Treppe (8) festlegbaren Basisprofil (9), wobei das Trittwinkelprofil (7) einen Trittschenkel (10) mit Abdeckflügel (11) und einen Anschlagschenkel (12) aufweist,
- einen lösbar und in seiner Lage anpassbar an der Profilkonstruktion (3) angeordneten, in Form einer Profilschiene ausgebildeten Wechseleinsatz (4), an dem zumindest an einem seiner beiden einander gegenüberliegenden Längsränder eine mit einer Profilierung (5) eines Randes (1) eines Bodenbelages (2) zusammenwirkbare Halterung (6) ausgebildet ist, die zumindest teilweise komplementär zur Profilierung (5) ausgebildet ist, wobei der Wechseleinsatz (4) mindestens eine Abstützrippe (15) aufweist, die an ihrem freien Stützrand (16) eine Rasterung (17') aufweist, die mit einer entsprechenden Rasterung (17") auf dem Basisprofil (9) zusammenwirkt, und wobei
- der Anschlagschenkel (12) mit einer Profilierung (12') versehen ist, die mit einer entsprechenden Profilierung (12") an einer Stirnkante des Basisprofils (9) zusammenwirkt,
**dadurch gekennzeichnet dass** das Trittwinkelprofil (7) an seiner Unterseite eine Anschlagrippe (27) aufweist, die mit einer an den Basisprofil (9) nach oben ragenden Gegenrippe (28) zur Anlage kommt, wobei zwischen der Anschlagrippe (27) und der Gegenrippe (28) eine Verrastung (29) ausgebildet ist.

2. Vorrichtung nach Anspruch 1, **dadurch gekennzeichnet, dass** der Wechseleinsatz (4) ganz oder zum Teil zwischen dem Trittschenkel (10) und dem Basisprofil (9) angeordnet ist und an seinen beiden einander gegenüberliegenden Längsrändern voneinander unterschiedliche Halterungen zum Einsatz an unterschiedlichen Profilierungen von Nut - FederVerbindungen aufweist.

3. Vorrichtung nach Anspruch 1 oder 2, **dadurch gekennzeichnet, dass** der Wechseleinsatz (4) an der Profilkonstruktion (3) mittels Verrastung (13) gehalten ist.

4. Vorrichtung nach Anspruch 3, **dadurch gekennzeichnet, dass** der Wechseleinsatz (4) als Teil der Verrastung im Bereich der komplementären Halterung (6) eine Verankerung (18) mit dem Basisprofil (9) und/oder dem Trittschenkel (10) aufweist.

5. Vorrichtung nach Anspruch 4, **dadurch gekennzeichnet, dass** die Abstützrippe (15) einen der Aufnahme waagerechter Kräfte dienenden Steg (14) aufweist, an den die komplementäre Halterung (6) anschließt.

6. Vorrichtung nach einem der vorangegangenen Ansprüche, dadurch genkennzeichnet, dass der Wechseleinsatz (4) als Stranggussprofil aus Aluminium oder Kunststoff gefertigt ist

7. Vorrichtung nach einem der vorangegangenen Ansprüche, **dadurch gekennzeichnet, dass** die Profilierung (5) in der Art einer Nut - FederVerbindung (19) ausgestaltet ist und mindestens einen zur Nut - FederEbene (20) hervortretenden Vorsprung (21) bzw. eine Vertiefung (22) aufweist.

8. Vorrichtung nach Anspruch 7, **dadurch gekennzeichnet, dass** die komplementäre Halterung (6) mindestens eine Halte - Rippe oder einen Halte - Vorsprung (23) aufweist, die die Nut - Feder - Verbindung (19) nach oben sichert und/oder den Vorsprung (21) zumindest zum Teil hintergreift bzw. in die Vertiefung (22) eingreift.

9. Vorrichtung nach Anspruch 8, **dadurch gekennzeichnet, dass** an der mindestens einen Halte Rippe (23) Sollbruchstellen (24) zur Höhenreduzierung ausgebildet sind.

10. Vorrichtung nach Anspruch 1 und 9, **dadurch gekennzeichnet, dass** der Wechseleinsatz (4) im Wesentlichen U-förmig ausgebildet ist und ein U-Schenkel als Halte-Rippe (23) und der andere U-Schenkel als Abstützrippe (15) ausgebildet ist.

11. Vorrichtung nach Anspruch 5 und 10, **dadurch gekennzeichnet, dass** die beiden Schenkel (23, 15) durch den Steg (14) miteinander verbunden sind und die jeweiligen Schenkel Sollbruchstellen (24) aufweisen.

12. Vorrichtung nach einem der vorangegangenen Ansprüche, **dadurch gekennzeichnet dass**, der Wechseleinsatz (4) mindestens eine Bohrung (25) aufweist, um den Wechseleinsatz (4) mittels eines Befestigungsmittels (26) an einen geraden Rand (1) eines Bodenbelags (2) festzulegen.

## Claims

1. Device comprising
- a profile construction configured as a step nosing profile (3) with an angular step profile (7) and a base profile (9) which can be secured on a step (8), wherein the angular step profile (7) has a step leg (10) with a covering wing (11) and a stop leg (12),
- an removable and in its position to the profile construction (3) adaptable interchangeable insert (4), on which, at least on one of its two mutually opposite longitudinal edges with a profiling (5) of an edge (1) of a floor covering (2) a cooperateable retainer (6) is configured, which is configured to be at least partly complementary to the profiling (5), wherein the interchangeable insert (4) comprises at least one supporting rib (15) which has on its free supporting edge (16) a locking engagement (17') which cooperates with a corresponding locking engagement (17") on the base profile (9), and wherein
- the stop leg (12) is provided with a profiling (12'), which cooperates with a corresponding profiling (12") on the leading edge of the base profile (9),
**characterized in that** the angular step profile (7) is provided on its lower side with a stop rib (27) which comes to rest against a counter rib (28) which projects upwards on the base profile (9), wherein a lock (29) is formed between the stop rib (27) and the counter rib (28).

2. Device according to claim 1, **characterised in that** the interchangeable insert (4) is completely or partly arranged between the step leg (10) and the base profile (9) and has on its two mutually opposite longitudinal edges different retainers to be used on different profilings of tongue and groove joints.

3. Device according to claim 1 or claim 2, **characterised in that** the interchangeable insert (4) is held on the profile construction (3) by means of a lock (13).

4. Device according to claim 3, **characterised in that** the interchangeable insert (4) has, as part of the lock in the region of the complementary retainer (6), an anchorage (18) with the base profile (9) and/or the step leg (10).

5. Device according to claim 4, **characterised in that** the supporting rib (15) has a web (14) which is used to absorb horizontal forces and which is adjoined by the complementary retainer (6).

6. Device according to any one of the preceding claims, **characterised in that** the interchangeable insert (4) is produced as a strand cast profile from aluminium or plastics material.

7. Device according to any one of the preceding claims, **characterised in that** the profiling (5) is configured as a tongue and groove joint (19) and has at least one projection (21) which is prominent relative to the tongue and groove plane (20), or has a recess (22).

8. Device according to claim 7, **characterised in that** the complementary retainer (6) has at least one retaining rib or one retaining projection (23) which upwardly secures the tongue and groove joint (19) and/or at least partly engages behind the projection (21) or engages into the recess (22).

9. Device according to claim 8, **characterised in that** predetermined breaking points (24) to reduce the height are formed on the at least one retaining rib (23).

10. Device according to claims 1 and 9, **characterised in that** the interchangeable insert (4) is configured substantially in a U shape and one leg of the U is configured as retaining rib (23) and the other leg of the U is configured as supporting rib (15).

11. Device according to claims 5 and 10, **characterised in that** the two legs (23, 15) are joined together by the web (14) and the respective legs have predetermined breaking points (24).

12. device according to any one of the preceding claims, **characterised in that** the interchangeable insert (4) has at least one hole (25) to secure the interchangeable insert (4) by an attachment means (26) to a straight edge (1) of a floor covering (2).

## Revendications

1. Dispositif comprenant
- une structure profilée réalisée sous la forme d'un profilé à arête en escalier (3) pourvu d'un profilé formant une cornière (7) et d'un profilé de base (9) pouvant être fixé sur un escalier (8), sachant que le profilé formant cornière (7) présente une branche (10) pourvue d'une aile de recouvrement (11) et une branche de butée (12),
- un insert interchangeable (4) disposé de manière amovible et de manière à pouvoir adapter sa position au niveau de la structure profilée (3), réalisé sous la forme d'un rail profilé, au niveau duquel un support (6) pouvant coopérer avec un profilage (5) d'un bord (1) d'un dallage (2) est réalisé au moins au niveau de l'un de ses deux bords longitudinaux se faisant face l'un l'autre, lequel support est réalisé au moins en partie de manière complémentaire au profilage (5), sachant que l'insert interchangeable (4) présente au moins une nervure d'appui (15), qui présente, au niveau de son bord de soutien (16) libre, un crantage (17'), qui coopère avec un crantage (17") correspondant sur le profilé de base (9),
- sachant que la branche de butée (12) est dotée d'un profilage (12'), qui coopère avec un profilage (12") correspondant au niveau d'une arête frontale du profilé de base (9),
**caractérisé en ce que** le profilé formant cornière (7) présente, au niveau de son côté inférieur, une nervure de butée (27), qui vient en appui avec une contre-nervure (28) dépassant vers le haut au niveau du profilé de base (9),sachant qu'un crantage d'assemblage (29) est réalisé entre la nervure de butée (27) et la contre-nervure (28).

2. Dispositif selon la revendication 1, **caractérisé en ce que** l'insert interchangeable (4) est disposé en totalité ou en partie entre la branche (10) et le profilé de base (9) et présente, au niveau de ses deux bords longitudinaux se faisant face l'un l'autre, des supports différents les uns des autres destinés à être utilisés au niveau de profilages différents de systèmes d'assemblages à rainure et à languette.

3. Dispositif selon la revendication 1 ou 2, **caractérisé en ce que** l'insert interchangeable (4) est maintenu au niveau de la structure profilée (3) au moyen d'un crantage d'assemblage (13).

4. Dispositif selon la revendication 3, **caractérisé en ce que** l'insert interchangeable (4) présente, en tant que partie du crantage d'assemblage, dans la zone du support (6) complémentaire, un ancrage (18) pourvu d'un profilé de base (9) et/ou de la branche (10).

5. Dispositif selon la revendication 4,
**caractérisé en ce que** la nervure d'appui (15) présente une entretoise (14) servant à absorber des forces horizontales, à laquelle se raccorde le support (6) complémentaire.

6. Dispositif selon l'une quelconque des revendications précédentes, **caractérisé en ce que** l'insert interchangeable (4) est fabriqué sous la forme d'un profilé coulé en continu composé d'aluminium ou de matière plastique.

7. Dispositif selon l'une quelconque des revendications précédentes, **caractérisé en ce que** le profilage (5) est configuré à la manière d'un assemblage à rainure et à languette (19) et présente au moins une partie faisant saillie (21) ou un renfoncement (22) faisant saillie en direction du plan de rainure et de languette (20).

8. Dispositif selon la revendication 7, **caractérisé en ce que** le support (6) complémentaire présente au moins une nervure de maintien ou une partie faisant saillie de maintien (23), qui bloque vers le haut l'assemblage à rainure et à languette (19) et/ou vient en prise par l'arrière au moins en partie avec la partie faisant saillie (21) ou vient en prise avec le renfoncement (22).

9. Dispositif selon la revendication 8, **caractérisé en ce que** des points de rupture théorique (24) servant à réduire la hauteur sont réalisés au niveau de l'au moins une nervure de maintien (23).

10. Dispositif selon la revendication 1 et 9, **caractérisé en ce que** l'insert interchangeable (4) est réalisé sensiblement de manière à présenter une forme de U, et **en ce qu'**une branche en U est réalisée sous la forme de la nervure de maintien (23) et l'autre branche en U est réalisée sous la forme de la nervure d'appui (15).

11. Dispositif selon la revendication 5 et 10, **caractérisé en ce que** les deux branches (23, 15) sont reliées l'une à l'autre par l'entretoise (14), et **en ce que** les branches respectives présentent des points de rupture théorique (24).

12. Dispositif selon l'une quelconque des revendications précédentes, **caractérisé en ce que** l'insert interchangeable (4) présente au moins un alésage (25) afin de fixer l'insert interchangeable (4) au moyen d'un moyen de fixation (26) au niveau d'un bord (1) rectiligne d'un dallage (2).
